# FASCICULE DE BREVET EUROPEEN

(11) **EP 2 879 621 B1**
(45) Date de publication et mention de la délivrance du brevet: **15.03.2017**
(21) Numéro de dépôt: 13815010.7
(22) Date de dépôt: 05.08.2013
(51) Int. Cl.: A61F 2/30

(54) **PROTHESE CONDYLIENNE POUR UNE ARTICULATION TEMPORO-MANDIBULAIRE**
KONDYLÄRPROTHESE FÜR EIN KIEFERGELENK
CONDYLAR PROSTHESIS FOR A TEMPORO-MANDIBULAR JOINT

(30) Priorité: 06.08.2012 FR 1257637
(43) Date de publication de la demande: 10.06.2015
(73) Titulaire: Université de Bordeaux, 33405 Talence (FR); Centre National de la Recherche Scientifique (C.N.R.S.), 75016 Paris (FR); Universidade De Aveiro, 3810-193 Aveiro (PT)
(72) Inventeur: RAMOS, Antonio, Ilhavo (PT); MESNARD, Michel, F-33200 Bordeaux (FR)
(74) Mandataire: Aquinov
(86) Numéro de dépôt international: PCT/FR2013/051882
(87) Numéro de publication internationale: WO 2014/023903

(56) Documents cités:
- EP-A1- 0 524 874
- WO-A1-97/11651
- FR-A1- 2 558 721
- US-A- 4 726 808
- US-A- 5 489 305
- US-A1- 2011 137 422

## Description

La présente invention est relative à une prothèse condylienne pour une articulation temporo-mandibulaire.
L'articulation temporo-mandibulaire bilatérale est une diarthrose enfermée dans une capsule articulaire et permettant de relier le condyle de la mandibule à la cavité ou fosse glénoïde de l'os temporal du crâne par l'intermédiaire d'un disque fibro-cartilagineux.
En outre, cette articulation est animée par différents muscles : muscles masticateurs, tel le muscle temporal, le muscle masséter, les muscles ptérygoïdiens, et les muscles sus-hyoïdiens et sous-hyoïdiens, afin d'obtenir les mouvements de propulsion ou de rétropulsion, d'abaissement ou d'élévation de la mandibule, et de diduction.
Anatomiquement, deux articulations fonctionnent en synergie de part et d'autre du crâne pour permettre les mouvements précités de la mandibule et remplir les fonctions de déglutition, salivaire, de mastication, de phonation, et de bâillement.
Comme pour d'autres articulations, les fonctions autorisées par l'articulation temporo-mandibulaire sont susceptibles d'être affectées à long terme par différentes pathologies ou plus brutalement suite à un traumatisme.
La pathologie entraînant le plus souvent une réduction des fonctions de cette articulation est l'arthrose, cette dégénérescence pouvant être causée ou aggravée par le stress actuellement constaté chez des personnes de plus en plus jeunes.
Pour donner un ordre d'idées, on estime que, chaque année aux Etats-Unis, plus d'un million de nouveaux patients souffrent de troubles de l'articulation temporo-mandibulaire, et que, parmi ces cas pathologiques, 2 à 3% imposent une reconstruction.
La dégénérescence de cette articulation peut aussi avoir pour origine une mauvaise occlusion dentaire, une parafonction consistant à serrer ou grincer des dents, une dysfonction dans le jeu des muscles agonistes et antagonistes, une réduction de la phase de repos physiologique des muscles, des crampes musculaires, ou une mauvaise position de la langue entrainant une mauvaise déglutition salivaire.
A un premier stade des dégradations des fonctions articulaires, les amplitudes des mouvements de la mandibule par rapport au crâne diminuent et seul le confort physique et/ou psychologique des patients est affecté.

Cependant, à un stade beaucoup plus élevé, il peut se produire une ossification tendant à être une cause d'invalidité car figeant complètement la mandibule dans la cavité glénoïde.
De plus, et dès le premier stade des dégradations, les personnes affectées peuvent ressentir des douleurs importantes.
Aussi, pour soulager les patients et stopper la dégénérescence de l'articulation, différentes solutions ont été développées.
Selon une première méthode de réhabilitation, une interface articulaire est réalisée lors d'une intervention chirurgicale en interposant entre le condyle mandibulaire et la fosse glénoïde une cupule, notamment fabriquée dans un biomatériau, ou un lambeau musculaire prélevé sur le patient.
Cette première méthode peut offrir des résultats satisfaisants dans certains cas. Cependant, le taux de récidive reste supérieur à 50% lorsque l'intervention est consécutive à une infection ou à une maladie dégénérative.
De plus, l'intervention chirurgicale reste techniquement délicate et la gestion des séquelles consécutives aux prélèvements effectués alourdit la période postopératoire.
Face aux échecs et aux inconvénients de cette première méthode de réhabilitation, l'implantation d'une prothèse constitue une meilleure solution, tant pour soigner les patients adultes que pour soigner les enfants, par exemple dans le cas d'un traumatisme irréversible et malgré une croissance inachevée.
Il existe différentes catégories de prothèses pour réhabiliter une articulation temporo-mandibulaire : les prothèses se substituant à la cavité glénoïde et à la pente temporale, les prothèses remplaçant le condyle mandibulaire, et les prothèses complètes pour le remplacement combiné de la surface articulaire temporale et du condyle.
La présente invention s'intéresse plus particulièrement au remplacement du condyle mandibulaire, mais elle concerne aussi de ce fait la conception des prothèses complètes.
La mise en place d'une prothèse est une opération complexe en raison de la nature des structures osseuses adjacentes à l'articulation temporo-mandibulaire.
Aussi, la géométrie et le matériau de la prothèse jouent un rôle prépondérant dans la qualité et la durée de vie de l'articulation prothétique.
Actuellement, seuls deux modèles de prothèses complètes de l'articulation temporo-mandibulaire sont disponibles sur le marché et approuvés par les autorités compétentes telle la Food and Drug Administration.

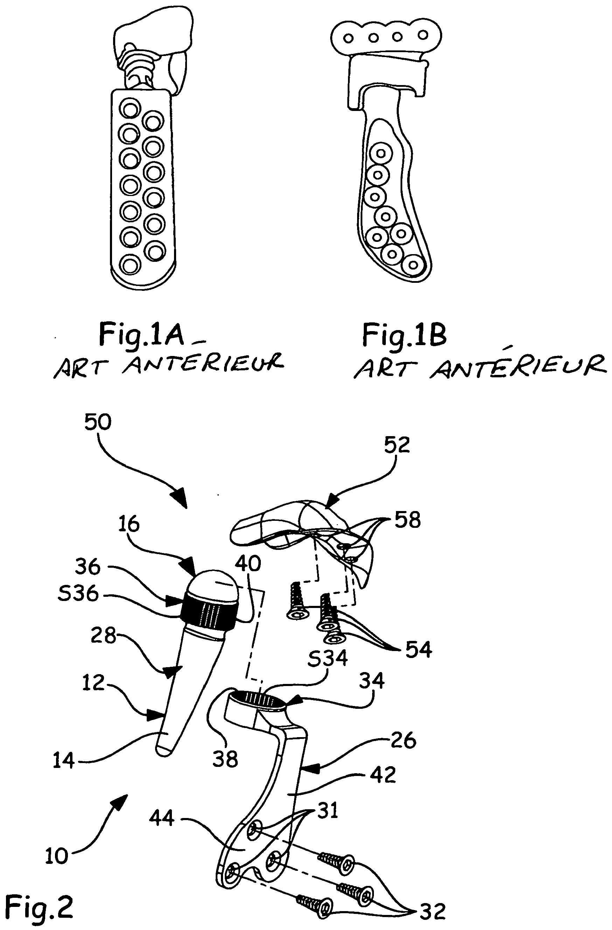

Dans ces deux modèles de prothèse, illustrés par les figures 1A et 1B, le condyle de substitution est supporté par un implant mandibulaire fixé sur la face latérale de la branche mandibulaire à l'aide de vis chirurgicales.
Comme l'illustrent les figures 1A et 1B, l'implant mandibulaire de ces prothèses de l'art antérieur comprend une plaque de fixation plane et massive sans formes réellement adaptées au profil osseux de la face latérale de la branche mandibulaire.
Par conséquent, l'implant mandibulaire n'est jamais intégralement en contact avec la face latérale de la branche mandibulaire, et l'épaisseur de l'implant lui confère des capacités mécaniques, telle la rigidité, bien supérieures à celles de la branche mandibulaire, ce qui accroît les risques de microdéplacements de la prothèse et de malocclusion.
D'un point de vue anatomique, cette conception massive de l'implant mandibulaire réduit le confort du patient, notamment par une gêne de l'insertion du muscle masséter fortement activé par la mastication.
De plus, d'un point de vue biomécanique, les différents orifices de maintien prévus dans l'implant mandibulaire ne permettent pas de distribuer correctement les vis lors de l'intervention chirurgicale, et notamment de manière à prendre en compte le déport de la charge exercée sur le condyle de substitution et à pouvoir résister au couple de torsion ainsi exercé sur ce dernier. Après plusieurs années d'utilisation de ces prothèses, on a constaté que le transfert de charge non optimisé vers les tissus osseux génère :
- une concentration de contraintes dans la zone proche du condyle et de la première vis, ainsi qu'une ostéointégration aléatoire dans la zone centrale,
- une augmentation de la rigidité de la mandibule prothésée et, par la suite, un risque élevé de dégénérescence des tissus osseux voisins,
- une réduction de la variation de la distance inter-condyles sous chargement et, par la suite, une perturbation de l'occlusion dentaire.
Ces effets négatifs sont susceptibles de causer à plus ou moins long terme divers désagréments aux patients.
Ces désagréments peuvent se traduire par une limitation de certaines mobilités de la mandibule, par des douleurs persistantes ou des phénomènes inflammatoires, ou, quelques fois, par une rupture de la prothèse ou de la liaison mandibulaire nécessitant une reprise chirurgicale et éventuellement la mise en place d'une nouvelle prothèse.
Aussi, d'un point de vue général, les différentes prothèses utilisées jusqûà aujourd'hui comprennent des implants mandibulaires aux conceptions empiriques, essentiellement fondées sur des données d'anatomie descriptive, et qui ne permettent pas de parvenir à des résultats satisfaisants en terme de qualité et de fiabilité dans le temps.
Une solution idéale serait de concevoir la prothèse et son implant mandibulaire en fonction de l'anatomie de chaque patient et des formes extérieures de sa mandibule.
Cette solution n'est évidemment pas viable en raison de l'importance des coûts et des moyens qui devraient être mobilisés pour l'analyse anatomique de la mandibule du patient, pour la fabrication individualisée de la prothèse et pour sa mise en place.
L'art antérieur le plus proche est représenté par EP-A-0524874.
La présente invention vise à pallier les différents inconvénients rencontrés avec les prothèses de l'art antérieur.
A cet effet, l'invention a pour objet une prothèse condylienne pour une articulation temporo-mandibulaire, la prothèse condylienne comprenant un implant mandibulaire, destiné à être solidarisé à la branche d'une mandibule, et une tête d'articulation, destinée à faire office de condyle mandibulaire de substitution, l'implant mandibulaire comprenant une queue d'ancrage permettant la mise en oeuvre d'une liaison intramédullaire dans le sommet de la branche de la mandibule, et l'extrémité distale de la queue d'ancrage étant déportée vers l'avant de la prothèse par rapport à la tête d'articulation et dans un plan médian de la prothèse.
Selon l'invention, la prothèse condylienne se caractérise en ce que la queue d'ancrage suit un profil courbe dans sa longueur et dans le plan médian de la prothèse, la direction de courbure de la queue d'ancrage étant orientée vers l'avant de la prothèse.
Avantageusement, la combinaison du déport de l'extrémité distale de la queue d'ancrage et de la courbure de la queue d'ancrage permet d'obtenir une liaison intramédullaire solide et durable dans le sommet de la branche de la mandibule du patient.
La conception de la prothèse condylienne selon l'invention, et plus particulièrement la queue d'ancrage permettant de mettre en oeuvre une liaison intra médullaire entre l'implant mandibulaire et les tissus osseux, autorise :
- une réduction de la résection osseuse nécessaire à la suppression du condyle naturel pathologique, ce qui facilite une éventuelle reprise chirurgicale,
- une diminution de la durée de l'intervention chirurgicale et une réduction des évaluations qualitatives laissées au chirurgien lors de la mise en position de l'implant mandibulaire et telles que la position angulaire de l'implant, le nombre, le type et la distribution des vis, etc,
- une suppression de l'effet de couple en situant l'ensemble de l'implant mandibulaire dans un plan para-sagittal, et en optant pour une tête à géométrie sphérique pour remplacer le condyle.

Plus généralement, la prothèse condylienne selon l'invention permet de résoudre les problèmes rencontrés avec les prothèses de l'art antérieur, de procurer au patient un réel confort et de répondre à un besoin médical en augmentation continue en raison du stress croissant dans la population jeune et d'un allongement simultané de la durée de vie.
La présente invention couvre aussi une prothèse complète pour une articulation temporo-mandibulaire comprenant une prothèse condylienne selon l'invention.
D'autres caractéristiques et avantages ressortiront de la description qui va suivre de l'invention, description donnée à titre d'exemple uniquement, en regard des dessins annexés sur lesquels :
- les figures 1A et 1B représentent des prothèses complètes selon l'art antérieur,
- la figure 2 représente une vue en perspective et en éclaté d'une prothèse complète comprenant une prothèse condylienne selon l'invention,
- la figure 3 représente une vue en perspective de la mise en place d'une prothèse selon l'invention sur une branche d'une mandibule humaine,
- la figure 4 représente une vue de côté d'une prothèse condylienne selon l'invention,
- la figure 5 représente une vue d'arrière d'une prothèse condylienne selon l'invention,
- la figure 6 représente une vue en coupe selon la ligne A-A de la vue d'arrière d'une prothèse condylienne selon l'invention représentée en figure 5, et
- la figure 7 représente une vue de côté d'une prothèse condylienne selon l'invention équipée avec une seconde variante d'une plaque de liaison extérieure,
- la figure 8 représente une vue de côté d'une prothèse complète avec une prothèse condylienne selon l'invention équipée avec une troisième variante d'une plaque de liaison extérieure,
- la figure 9 représente une vue de côté en perspective et en éclaté d'une prothèse complète comprenant une prothèse condylienne dans un mode réalisation optimisé selon l'invention,
- la figure 10 représente une vue de dessous en perspective et en éclaté d'une prothèse complète comprenant une prothèse condylienne dans un mode réalisation optimisé selon l'invention.

La présente invention est relative à une prothèse condylienne 10 pour une articulation temporo-mandibulaire telle qu'elle est illustrée en figure 2.
De façon connue, cette prothèse condylienne 10 comprend un implant mandibulaire 12 destiné à être solidarisé à la branche d'une mandibule humaine, ainsi qu'une tête d'articulation 16 destinée à faire office de condyle mandibulaire de substitution.

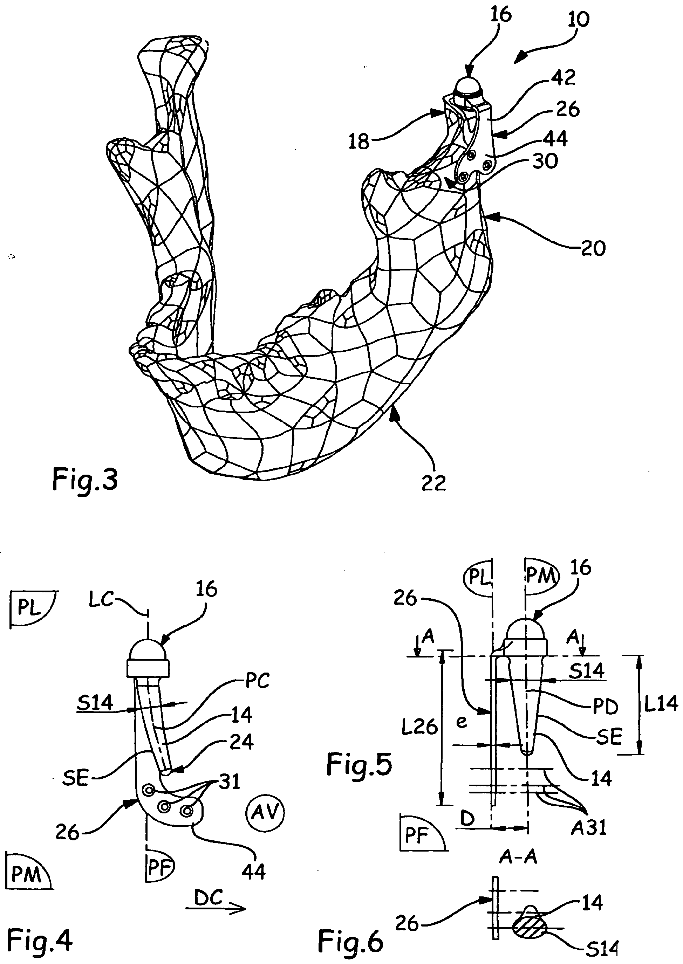

Selon l'invention et comme l'illustre la figure 3, l'implant mandibulaire 12 comprend une queue d'ancrage 14 permettant la mise en oeuvre d'une liaison intramédullaire dans le sommet 18 de la branche 20 de la mandibule 22.
Grâce à cette liaison intramédullaire, et comparativement aux prothèses de l'art antérieur, le transfert de charge de la tête d'articulation 16 vers les tissus osseux de la mandibule 22 n'est pas déporté, et par conséquent, la tête d'articulation 16 ne subit pas de couple de torsion dû à la solidarisation de l'implant 12 à la mandibule 22.
Pour la suite de la description, et en regard de l'orientation de la prothèse 10 sur les différentes figures, le plan médian PM de la prothèse 10 est un plan vertical contenant l'axe longitudinal central LC de la prothèse et séparant l'implant mandibulaire 12 en deux parties droite et gauche. Et, le plan frontal PF de la prothèse 10 est un plan vertical contenant l'axe longitudinal central LC de la prothèse, perpendiculaire au plan médian PM, et séparant l'implant mandibulaire 12 en deux parties avant et arrière.
Toujours dans l'objectif d'améliorer le transfert de charge de la tête d'articulation 16 vers les tissus osseux de la mandibule 22, et comme illustré par la figure 4, l'extrémité distale 24 de la queue d'ancrage 14 est déportée vers l'avant AV de la prothèse 10 par rapport à la tête d'articulation 16 et dans le plan médian PM de la prothèse 10.
Dans un mode de réalisation préféré de la queue d'ancrage 14 visant à mieux répartir les efforts dans la branche mandibulaire 20 et à éviter les zones non sollicitées, la queue d'ancrage 14 suit un profil courbe PC dans sa longueur L14 et dans le plan médian PM de la prothèse, la direction de courbure DC de la queue d'ancrage 14 étant orientée vers l'avant AV de la prothèse. Avantageusement, cette courbure de la queue d'ancrage 14 permet aussi d'éviter une rotation de l'implant mandibulaire 12 par rapport à la branche mandibulaire 20 une fois celui-ci ancré dans cette dernière.
Toujours dans un mode de réalisation préféré de la queue d'ancrage 14 et afin de maintenir les efforts issus de la tête d'articulation 16 sensiblement dans le plan paramédian de la branche mandibulaire 20, la queue d'ancrage 14 suit un profil droit PD dans sa longueur L14 et dans le plan frontal PF de la prothèse perpendiculaire au plan médian PM, comme l'illustre la figure 5.
Afin de faciliter l'insertion de la queue d'ancrage 14 dans le trou d'ancrage réalisé au sommet 18 de la branche mandibulaire 20 lors de l'intervention chirurgicale et de pouvoir l'ancrer légèrement en force dans ce trou, la section S14 de la queue d'ancrage 14 réduit dans sa longueur L14 et vers son extrémité distale 24.

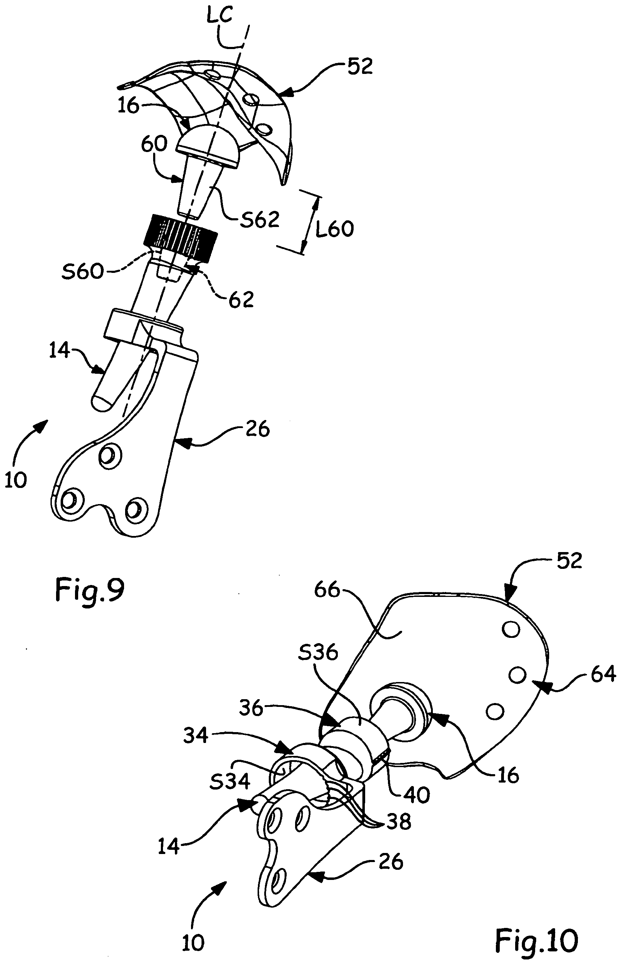

Comme l'illustrent les figures 4 et 5, la section S14 de la queue d'ancrage 14 réduit, de préférence progressivement, dans sa longueur L14 à la fois dans le plan médian PM et dans le plan frontal PF.
Dans l'objectif de favoriser un maintien mécanique de l'implant mandibulaire 12 dans le trou d'ancrage réalisé dans la branche mandibulaire 20, et comme l'illustre la vue en coupe en figure 6, la section S14 de la queue d'ancrage 14 est de préférence de type elliptique dans toute ou partie de sa longueur L14.
En raison de l'anatomie de la branche mandibulaire 20, le grand axe de l'ellipse reste sensiblement parallèle au plan frontal PF de la prothèse dans la longueur L14 de la queue d'ancrage 14, tandis que le petit axe de l'ellipse reste sensiblement parallèle au plan médian PM de la prothèse dans la longueur L14 de la queue d'ancrage 14.
Dans un mode de réalisation préféré de la queue d'ancrage 14, la queue d'ancrage 14 est de type elliptique sur toute sa longueur L14 et son extrémité distale 24 est de forme arrondie, et de préférence sphérique.
De manière générale, l'invention couvre une prothèse 10 avec une queue d'ancrage 14 de section S14 non-circulaire dans toute ou partie de sa longueur L14, toute forme non circulaire permettant à la section S14 d'empêcher la rotation et donc de favoriser le maintien de l'implant mandibulaire 12 dans la branche mandibulaire 20.
En vue de la mise en oeuvre de l'ancrage, la queue d'ancrage 14 présente une surface extérieure SE rugueuse, et de préférence granuleuse, traitée et non représentée sur les figures, pour augmenter la surface de liaison intramédullaire avec les tissus osseux de la branche mandibulaire 20 et pour favoriser l'ostéointégration de l'implant mandibulaire 12 dans la branche mandibulaire 20.
Cette surface extérieure SE rugueuse ou granuleuse favorise l'ancrage mécanique et biologique de l'implant 12 dans la branche mandibulaire, 20.
Avantageusement, les bris d'os issus du montage en force de la queue d'ancrage 14 viennent aussi renforcer l'ancrage biologique de l'implant 12, et cela en plus des enrobages spécifiques, notamment à base d'hydroxyapatite, utilisés pour favoriser la reconstruction osseuse.
La rugosité ou l'aspect granuleux de la surface extérieure SE de la queue d'ancrage 14 peuvent être obtenus par différents procédés mécaniques, tel un sablage, ou par des attaques chimiques, ces procédés étant connus de l'homme du métier.
Comme l'illustre la figure 9, dans un mode de réalisation optimisé de la prothèse 10 selon l'invention, le corps 28 de l'implant mandibulaire 12 est réalisé en deux parties, la tête d'articulation 16 étant rapportée sur la queue d'ancrage 14 de l'implant mandibulaire 12.

A cet effet, la tête d'articulation 16 comprend une queue d'emmanchement 60 et la queue d'ancrage 14 comprend un logement de réception 62 de cette queue d'emmanchement 60.
Plus en détails, la queue d'emmanchement 60 s'étend sous la tête d'articulation 16, et le logement de réception 62 est creusé dans la queue d'ancrage 14 le long de l'axe longitudinal central LC de la prothèse.
Grâce à cette réalisation en deux parties de l'implant mandibulaire, 12, un jeu de différentes têtes d'articulation 16 avec des queues d'emmanchement 60 de différentes longueurs L60 peut être fourni au chirurgien afin que ce dernier choisisse la tête 16 avec la longueur d'emmanchement L60 la plus adaptée à l'anatomie du patient.
Selon un premier avantage, lors d'une opération au cours de laquelle une importante partie du sommet 18 de la branche mandibulaire 20 doit être retirée car cette partie est malade ou trop endommagée, le chirurgien peut choisir une tête d'articulation 16 avec une queue d'emmanchement 60 plus longue, la longueur L60 de la queue d'emmanchement 60 permettant de compenser la perte osseuse.
Selon un autre avantage, lors d'une opération de reprise chirurgicale, par exemple due à la croissance de la branche mandibulaire 20 du patient, le chirurgien peut uniquement changer la tête d'articulation 16 en place et la remplacer par une tête d'articulation 16 avec une queue d'emmanchement 60 plus longue. Ainsi, le chirurgien peut facilement adapter la prothèse 10 à l'anatomie mandibulaire du patient sans retirer la queue d'ancrage 14 en place, et sans solliciter à nouveau les tissus osseux de la branche mandibulaire 20 du patient.
Selon un dernier avantage, grâce à cette réalisation en deux parties de l'implant mandibulaire 12, la tête d'articulation 16 peut être fabriquée dans un matériau permettant de réduire les frottements, comme par exemple la céramique.
En vue de faciliter l'assemblage de la tête d'articulation 16 avec la queue d'ancrage 14 et d'éviter une liaison vissée ou collée, le logement de réception 62 est conique.
Dans une variante préférée de l'assemblage de la tête d'articulation 16 avec la queue d'ancrage 14, la queue d'emmanchement 60 et le logement de réception 62 sont coniques, et leurs surfaces coniques d'emmanchement 560 et 562 correspondent l'une à l'autre.
Ainsi, lors d'une opération, le chirurgien doit juste procéder à un léger montage en force de la queue d'emmanchement 60 dans le logement de réception 62 pour assembler définitivement la tête d'articulation 16 à la queue d'ancrage 14.
Dans un mode de réalisation minimal de la prothèse selon l'invention, la queue d'ancrage 14 qui vient d'être décrite peut suffire à assurer la solidarisation de l'implant 12 à la branche mandibulaire, 20.

Cependant, la liaison intramédullaire entre l'implant 12 et les tissus osseux de la branche mandibulaire 20 n'étant pas immédiate, le patient devra observer une période de convalescence, d'autant plus longue que le patient est âgé, après l'opération chirurgicale.
Aussi, dans un mode de réalisation optimisé de la prothèse selon l'invention, la liaison intramédullaire permise par la queue d'ancrage 14 est complétée par une liaison extérieure sur la mandibule 22, cette liaison extérieure étant conçue de manière à être la moins invasive possible et permettant un maintien de la prothèse 10 pendant la période de convalescence et de reconstruction des tissus osseux autour de l'ancrage.
A cet effet, et comme illustré sur les différentes figures, l'implant mandibulaire 12 comprend aussi une plaque de liaison 26 extérieure solidaire du corps 28 de la prothèse 10 formé par la queue d'ancrage 14 et la tête d'articulation 16.
Cette plaque de liaison 26 étant destinée à être fixée sur la face latérale 30 de la branche mandibulaire 20 à l'aide d'au moins deux, et de préférence trois, vis chirurgicales 32, la plaque de liaison 26 comprend au moins deux, et de préférence, trois perçages 31 débouchants pour le passage des vis chirurgicales 32.
De préférence, la plaque de liaison 26 est formable afin de pouvoir être adaptée au mieux à l'anatomie osseuse de la mandibule du patient.
L'utilisation d'au moins deux vis chirurgicales 32 permet d'éviter un basculement ou une torsion de la tête d'articulation 16 autour de l'axe longitudinal central LC de la prothèse.
Afin d'éviter de fragiliser les tissus osseux autour de l'ancrage, ou de trop les solliciter, il est prévu de décaler les vis 32 par rapport à la queue d'ancrage 14.
A cet effet, les perçages 31 débouchants sont réalisés autour d'axes A31 déportés par rapport à la queue d'ancrage 14 tant dans le plan frontal PF de la prothèse que dans le plan médian PM de la prothèse.
Toutefois, les perçages 31 débouchants ne sont pas trop éloignés de la queue d'ancrage 14, de seulement quelques millimètres pour donner un ordre d'idées, ceci afin d'éviter un écart trop important par rapport au point de charge constitué par la tête d'articulation 16.
Dans un mode de réalisation préféré de la prothèse 10 visant à permettre la mise en place de l'ancrage dans un premier temps et de la fixation extérieure dans un deuxième temps, la plaque de liaison 26 extérieure est reliée de manière amovible au corps 28 de la prothèse 10. Avantageusement, cette liaison amovible laisse aussi le choix au chirurgien d'utiliser ou non la plaque de liaison 26 extérieure.
Dans une variante préférée de réalisation de cette liaison amovible, la plaque de liaison 26 extérieure comprend une partie de type annulaire 34 de solidarisation au corps 28 de la prothèse, et le corps 28 de la prothèse comprend une portée cylindrique 36 de réception de la partie annulaire 34 de la plaque de liaison 26 extérieure.
Plus en détails, la plaque de liaison 26 s'étend sous la partie annulaire 34, et la portée cylindrique 36 se situe entre la tête d'articulation 16 et la queue d'ancrage 14.
Afin d'immobiliser la plaque de liaison 26 extérieure en rotation autour du corps 28 de la prothèse, la surface extérieure 536 de la portée cylindrique 36 et la surface intérieure S34 de la partie annulaire 34 comprennent au moins une empreinte creuse 38 et une forme en relief 40 susceptible de coopérer l'une avec l'autre.
Selon une première variante illustrée en figure 2, la portée cylindrique 36 et la surface intérieure S34 de la partie annulaire 34 comprennent une pluralité d'empreintes creuses 38 et de formes en relief 40 pour permettre au chirurgien d'orienter la plaque de liaison 26 extérieure autour du corps 28 de la prothèse de la manière la plus adaptée à l'anatomie mandibulaire du patient.
Selon une autre variante illustrée en figure 10 et visant à simplifier la fabrication et à réduire les coûts de fabrication, la portée cylindrique 36 et la surface intérieure S34 de la partie annulaire 34 comprennent quelques empreintes creuses 38, cinq ou six de préférence, et une unique forme en relief 40 pour orienter et bloquer la plaque de liaison 26 extérieure en rotation autour du corps 28 de la prothèse.
Dans l'une ou l'autre des variantes précédentes, la (ou les) empreinte(s) creuse(s) 38 est (sont) prévue(s) dans la surface intérieure 534 de la partie annulaire 34 et la (ou les) forme(s) en relief 40 sont prévue(s) sur la surface extérieure S36 de la portée cylindrique 36.
Toujours dans l'une ou l'autre des variantes précédentes, la ou les empreintes creuses 38 et la ou les formes en relief 40 sont obtenues par moletage, par usinage d'une liaison de type languette rainure, etc.
Bien entendu, afin de se positionner au-delà de l'épaisseur des tissus osseux du sommet 18 de la branche mandibulaire, 20 préparés pour recevoir la prothèse 10 selon l'invention, la plaque de liaison 26 extérieure se situe dans un plan latéral PL déporté d'une distance D non nulle par rapport au plan médian PM de la prothèse dans le plan frontal PF de la prothèse.
Grâce à la pluralité d'empreintes creuses 38 et de formes en relief 40, le plan latéral PL parallèle peut être disposé parallèlement au plan médian PM, ou le chirurgien peut choisir d'incliner légèrement le plan latéral PL de la plaque de liaison 26 par rapport au plan médian PM de la prothèse si cela convient mieux à l'anatomie mandibulaire, du patient.
Pour obtenir un décalage des vis 32 par rapport à la queue d'ancrage 14, la plaque de liaison 26 extérieure peut prendre sensiblement la forme d'un L.

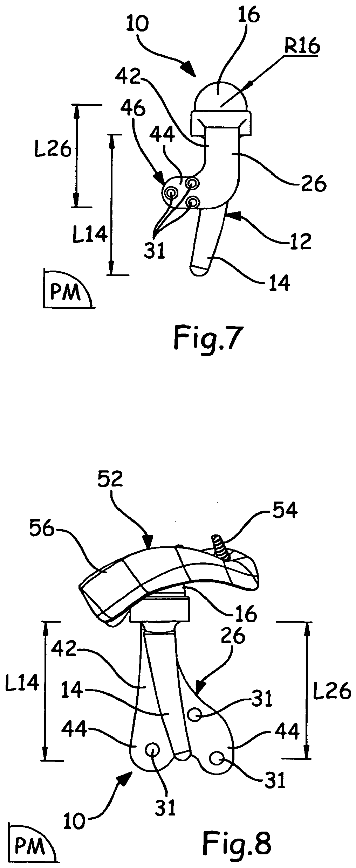

Plus précisément, la branche verticale 42 du L formée par la plaque de liaison 26 se prolongeant depuis la tête d'articulation 16 sensiblement dans la même direction que la queue d'ancrage 14, et la branche horizontale 44 du L formé par la plaque de liaison 26 se prolongeant dans la direction de courbure DC de la queue d'ancrage 14 dans un plan médian PM de la prothèse, les perçages débouchants 31 se situent dans la branche horizontale du L.
Dans une première variante de réalisation de la plaque de liaison 26 extérieure illustrée sur les figures 3 à 6, la longueur L26 de la plaque de liaison 26 est supérieure à la longueur L14 de la queue d'ancrage 14, et la branche horizontale 44 du L, et donc les perçages débouchants 31, sont déportés en dessous de la queue d'ancrage 14.
Dans une seconde variante de réalisation de la plaque de liaison 26 extérieure illustrée sur la figure 7, la longueur L26 de la plaque de liaison 26 est inférieure ou égale à la longueur L14 de la queue d'ancrage 14, et les perçages débouchants 31 sont réalisés dans l'extrémité 46 de la branche horizontale 44 du L s'étendant en devant de la queue d'ancrage 14.
Dans une troisième variante de réalisation de la plaque de liaison 26 extérieure illustrée sur les figures 2 et 8, la longueur L26 de la plaque de liaison 26 étant sensiblement égale à la longueur L14 de la queue d'ancrage 14, la branche horizontale 44 du L s'étend en devant et en arrière de la queue d'ancrage 14, et les perçages débouchants 31 sont réalisés dans la branche horizontale 44 du L de manière à être déportés en devant et en arrière de la queue d'ancrage 14.
De façon connue, et tant dans la conception de la plaque de liaison 26 que dans celle de la prothèse 10, les angles et les extrémités des différentes parties présentent des angles arrondis ou chanfreinés et/ou des extrémités arrondies.
Afin de limiter l'aspect invasif de la plaque formable de liaison 26, notamment vis-à-vis du muscle masséter, l'épaisseur e de la plaque de liaison 26 est inférieure à 0,8 millimètres.
Cette finesse de la plaque de liaison 26 permet aussi d'éviter une importante résection osseuse pour la mise en place de la prothèse 10, ce qui limite la quantité d'os enlevée et laisse donc la possibilité d'une reprise chirurgicale.
Avantageusement, et comme indiqué précédemment, la finesse de la plaque de liaison 26 permet aussi au chirurgien de la déformer pour épouser au mieux l'anatomie osseuse de la mandibule du patient.
Parallèlement, la longueur L14 de la queue d'ancrage 14 se situe aux alentours de 30 millimètres, et la longueur L26 de la plaque de liaison 26 varie entre 12 et plus de 30 millimètres suivant sa variante de réalisation.

Dans un mode de réalisation préféré visant à maîtriser le point de contact, et donc de charge, du condyle de substitution avec l'élément prothétique qui remplace le disque f ibro-cartilagineux, la queue d'ancrage 14 est surmontée par une tête d'articulation 16 sphérique.
Cette tête d'articulation 16 sphérique est centrée autour de l'axe longitudinal central LC de la prothèse et elle présente un rayon situé entre 3 et 4,5 millimètres.
La présente invention couvre aussi une prothèse complète 50 pour une articulation temporo-mandibulaire intégrant une prothèse condylienne 10 telle qu'elle vient d'être décrite.
Une telle prothèse complète 50, illustrée par les figures 2 et 8, comprend une prothèse temporale 52 destinée à être fixée au crâne, par exemple à l'aide de vis chirurgicales 54, et apte à recevoir la tête d'articulation 16 de la prothèse condylienne 10.
A cet effet, la prothèse temporale 52 prend la forme d'une plaque 56 à profil anatomique, d'épaisseur uniforme, et comportant différents perçages 58.
Cinématiquement, le profil de la plaque 56 permet de faire transiter, au frottement très réduit près, les efforts par le centre de la tête d'articulation 16 faisant office de néocondyle, et autorise des glissements de la tête d'articulation 16 par rapport à la prothèse temporale 52, et cela tant dans le plan frontal PF que dans le plan médian PM.
Avantageusement, le profil anatomique de la plaque 56 est choisi parmi un panel représentatif de 3 à 5 profils anatomiques déterminé à l'aide d'une étude statistique.
Ainsi, on évite la livraison de plusieurs dizaines de coquilles temporales avec chaque prothèse complète, et les quelques profil anatomiques choisis permettent à la prothèse temporale 52 de s'adapter aux patients tout en se rapprochant du profil anatomique de chacun.
De préférence, les perçages 58 sont localisés dans la plaque 56 de manière à permettre une implantation des vis 54 dans la zone osseuse la plus épaisse située à proximité de la fosse glénoïde.
Aussi, le crâne comprenant deux articulations, droite et gauche, la conception de la prothèse condylienne 10 pour une articulation temporo-mandibulaire droite ou gauche est identique, seule la plaque de liaison 26 extérieure devant être échangée contre un modèle symétrique par rapport au plan médian PM pour pouvoir adapter la prothèse 10 à l'autre articulation.
Avantageusement, et de préférence dans le cas où la tête d'articulation 16 est fabriquée dans un matériau permettant de réduire les frottements, la surface inférieure 64 de la prothèse temporale 52 sur laquelle la tête d'articulation 16 vient en appui est recouverte, au moins en partie, par un revêtement 66 d'un matériau permettant aussi de réduire les frottements, comme par exemple la céramique.

En réduisant les frottements entre la tête d'articulation 16 de l'implant 12 et la prothèse temporale 52, on limite les vibrations et donc le bruit dans la tête du patient, ce qui se traduit par un meilleur confort physiologique pour ce dernier.

## Revendications

1. Prothèse condylienne (10) pour une articulation temporo-mandibulaire, la prothèse condylienne (10) comprenant un implant mandibulaire (12), destiné à être solidarisé à la branche (20) d'une mandibule (22), et une tête d'articulation (16), destinée à faire office de condyle mandibulaire de substitution, l'implant mandibulaire (12) comprenant une queue d'ancrage (14) permettant la mise en oeuvre d'une liaison intramédullaire dans le sommet (18) de la branche (20) de la mandibule (22), et l'extrémité distale (24) de la queue d'ancrage (14) étant déportée vers l'avant (AV) de la prothèse (10) par rapport à la tête d'articulation (16) et dans un plan médian (PM) de la prothèse (10), la prothèse condylienne (10) étant **caractérisée en ce que** la queue d'ancrage (14) suit un profil courbe (PC) dans sa longueur (L14) et dans le plan médian (PM) de la prothèse, la direction de courbure (DC) de la queue d'ancrage (14) étant orientée vers l'avant (AV) de la prothèse.

2. Prothèse condylienne (10) selon la revendication précédente, dans laquelle la queue d'ancrage (14) suit un profil droit (PD) dans sa longueur (L14) et dans un plan frontal (PF) de la prothèse perpendiculaire au plan médian (PM).

3. Prothèse condylienne (10) selon l'une des revendications précédentes, dans laquelle la section (S14) de la queue d'ancrage (14) réduit dans sa longueur (L14) et vers son extrémité distale (24).

4. Prothèse condylienne (10) selon l'une des revendications précédentes, dans laquelle la section (S14) de la queue d'ancrage (14) est non circulaire dans toute ou partie de sa longueur (L14).

5. Prothèse condylienne (10) selon la revendication précédente, dans laquelle la section (S14) de la queue d'ancrage (14) est de type elliptique dans toute ou partie de sa longueur (L14), le grand axe de l'ellipse restant parallèle au plan frontal (PF) de la prothèse dans la longueur (L14) de la queue d'ancrage (14), et le petit axe de l'ellipse restant parallèle au plan médian (PM) de la prothèse dans la longueur (L14) de la queue d'ancrage (14).

6. Prothèse condylienne (10) selon l'une des revendications précédentes, dans laquelle la queue d'ancrage (14) présente une surface extérieure (SE) rugueuse ou granuleuse pour augmenter la surface de liaison intramédullaire et favoriser l'ostéointégration de l'implant mandibulaire (12) dans la branche mandibulaire (20).

7. Prothèse condylienne (10) selon l'une des revendications précédentes, dans laquelle l'implant mandibulaire (12) comprend aussi une plaque de liaison (26) extérieure solidaire du corps (28) de la prothèse (10) formé par la queue d'ancrage (14) et la tête d'articulation (16), cette plaque de liaison (26) extérieure comprenant au moins deux perçages (31) débouchants pour le passage de vis chirurgicales (32) et réalisés autour d'axes (A31) déportés par rapport à la queue d'ancrage (14) tant dans le plan frontal (PF) de la prothèse que dans le plan médian (PM) de la prothèse.

8. Prothèse condylienne (10) selon la revendication précédente, dans laquelle la plaque de liaison (26) extérieure est formable.

9. Prothèse condylienne (10) selon l'une des deux revendications précédentes, dans laquelle la plaque de liaison (26) extérieure est reliée de manière amovible au corps (28) de la prothèse (10).

10. Prothèse condylienne (10) selon la revendication précédente, dans laquelle la plaque de liaison (26) extérieure comprend une partie de type annulaire (34) de solidarisation au corps (28) de la prothèse, et dans laquelle le corps (28) de la prothèse comprend une portée. cylindrique (36) de réception de la partie annulaire (34) de la plaque de liaison (26) extérieure, la plaque de liaison (26) s'étendant sous la partie annulaire (34), la portée cylindrique (36) se situant entre la tête d'articulation (16) et la queue d'ancrage (14), et la surface extérieure (S36) de la portée cylindrique (36) et la surface intérieure (534) de la partie annulaire (34) comprenant au moins une empreinte creuse (38) et une forme en relief (40) susceptible de coopérer l'une avec l'autre pour immobiliser la plaque de liaison (26) extérieure en rotation autour du corps (28) de la prothèse.

11. Prothèse condylienne (10) selon l'une des quatre revendications précédentes, dans laquelle la plaque de liaison (26) extérieure se situe dans un plan latéral (PL) déporté d'une distance (D) non nulle par rapport au plan médian (PM) de la prothèse dans le plan frontal (PF) de la prothèse.

12. Prothèse condylienne selon l'une des cinq revendications précédentes, dans laquelle, la plaque de liaison (26) extérieure prenant sensiblement la forme d'un L dont la branche verticale (42) se prolonge depuis la tête d'articulation (16) sensiblement dans la même direction que la queue d'ancrage (14), et dont la branche horizontale (44) se prolonge dans la direction de courbure (DC) de la queue d'ancrage (14) dans un plan médian (PM) de la prothèse, les perçages débouchants (31) se situent dans la branche horizontale (44) du L.

13. Prothèse condylienne (10) selon la revendication précédente, dans laquelle la longueur (L26) de la plaque de liaison (26) est supérieure à la longueur (L14) de la queue d'ancrage (14), et dans laquelle la branche horizontale (44) du L, et donc les perçages débouchants (31), sont déportés en dessous de la queue d'ancrage (14).

14. Prothèse condylienne (10) selon la revendication 12, dans laquelle la longueur (L26) de la plaque de liaison (26) est inférieure ou égale à la longueur (L14) de la queue d'ancrage (14), et dans laquelle les perçages débouchants (31) sont réalisés vers l'extrémité (46) de la branche horizontale (44) du L s'étendant en devant de la queue d'ancrage (14).

15. Prothèse condylienne (10) selon la revendication 12, dans laquelle, la longueur (L26) de la plaque de liaison (26) étant sensiblement égale à la longueur (L14) de la queue d'ancrage (14), la branche horizontale (44) du L s'étend en devant et en arrière de la queue d'ancrage (14), et les perçages débouchants (31) sont réalisés dans la branche horizontale (44) du L de manière à être déportés en devant et en arrière de la queue d'ancrage (14).

16. Prothèse condylienne (10) selon l'une des revendications 7 à 15, dans laquelle l'épaisseur (e) de la plaque de liaison (26) est inférieure à 0,8 millimètres.

17. Prothèse condylienne (10) selon l'une des revendications précédentes, dans laquelle la queue d'ancrage (14) est surmontée par une tête d'articulation (16) sphérique.

18. Prothèse condylienne (10) selon l'une des revendications précédentes, dans laquelle le corps (28) de l'implant mandibulaire (12) est réalisé en deux parties, la tête d'articulation (16) étant rapportée sur la queue d'ancrage (14) de l'implant mandibulaire (12).

19. Prothèse condylienne (10) selon la revendication précédente, dans laquelle la tête d'articulation (16) comprend une queue d'emmanchement (60), et dans laquelle la queue d'ancrage (14) comprend un logement de réception (62) de cette queue d'emmanchement (60).

20. Prothèse complète (50) pour une articulation temporo-mandibulaire, **caractérisée en ce qu'**elle comprend une prothèse temporale (52) et une prothèse condylienne (10) selon l'une des revendications précédentes.

21. Prothèse complète (50) selon la revendication précédente, dans laquelle la tête d'articulation (16) est fabriquée dans un matériau permettant de réduire les frottements, et dans laquelle la surface inférieure (64) de la prothèse temporale (52) est recouverte, au moins en partie, par un revêtement (66) d'un matériau permettant de réduire les frottements.

## Patentansprüche

1. Kondylärprothese (10) für ein Kiefergelenk, wobei die Kondylärprothese (10) ein Kieferimplantat (12) aufweiset, das dazu bestimmt ist, mit dem Ast (20) eines Kiefers (22) verbunden zu werden, und einen Gelenkkopf (16) aufweist, der dazu bestimmt ist, als ersatzweiser mandibularer Kondylus zu dienen, wobei das Kieferimplantat (12) einen Verankerungsstift (14) aufweist, der die Bewerkstelligung einer intramedullären Verbindung in der Spitze (18) des Astes (20) des Kiefers (22) gestattet, und wobei das distale Ende (24) des Verankerungsstifts (14) bezüglich des Gelenkkopfes (16) in einer medialen Ebene (PM) der Prothese (10) zu einem vorderen Teil (AV) der Prothese (10) versetzt ist, wobei die Kondylärprothese (10) **dadurch gekennzeichnet ist, dass** der Verankerungsstift (14) über seine Länge (L14) hinweg und in einer medialen Ebene (PM) der Prothese einem gekrümmten Profil (PC) folgt, wobei die Krümmungsrichtung (DC) des Verankerungsstifts (14) zu dem vorderen Teil (AV) der Prothese ausgerichtet ist.

2. Kondylärprothese (10) nach dem vorhergehenden Anspruch, bei der der Verankerungsstift (14) über eine Länge (L14) hinweg und in einer frontalen Ebene (PF) der Prothese, die im rechten Winkel zur medialen Ebene (PM) steht, einem geraden Profil (PD) folgt.

3. Kondylärprothese (10) nach einem der vorhergehenden Ansprüche, bei der sich der Querschnitt (S14) des Verankerungsstifts (14) über seine Länge (L14) hinweg und zu seinem distalen Ende (24) hin verjüngt.

4. Kondylärprothese (10) nach einem der vorhergehenden Ansprüche, bei der der Querschnitt (S14) des Verankerungsstifts (14) über seine gesamte oder einen Teil seiner Länge (L14) hinweg nicht kreisförmig ist.

5. Kondylärprothese (10) nach dem vorhergehenden Anspruch, bei der der Querschnitt (S14) des Verankerungsstifts (14) über seine gesamte oder einen Teil seiner Länge (L14) von der elliptischen Art ist, wobei die große Achse der Ellipse über die Länge (L14) des Verankerungsstifts (14) hinweg parallel zur frontalen Ebene (PF) bleibt und die kleine Achse der Ellipse über die Länge (L14) des Verankerungsstifts (14) hinweg parallel zur medialen Ebene (PM) der Prothese bleibt.

6. Kondylärprothese (10) nach einem der vorhergehenden Ansprüche, bei der der Verankerungsstift (14) eine raue oder körnige äußere Oberfläche (SE) aufweist, um die intramedulläre Verbindungsfläche zu vergrößern und die Osteointegration des Kieferimplantats (12) in dem mandibularen Ast (20) zu fördern.

7. Kondylärprothese (10) nach einem der vorhergehenden Ansprüche, bei der das Kieferimplantat (12) auch eine äußere Verbindungsplatte (26) aufweist, die am Körper (28) der Prothese (10) angebracht ist, die von dem Verankerungsstift (14) und dem Gelenkkopf (16) gebildet ist, wobei die äußere Verbindungsplatte (26) wenigstens zwei Durchführungen (31) aufweist, die zum Hindurchführen von chirurgischen Schrauben (32) dienen und die um Achsen (A31) ausgebildet sind, die in der frontalen Ebene (PF) ebenso wie in der medialen Ebene (PM) der Prothese bezüglich des Verankerungsstifts (14) versetzt sind.

8. Kondylärprothese (10) nach dem vorhergehenden Anspruch, bei der die äußere Verbindungsplatte (26) verformbar ist.

9. Kondylärprothese (10) nach einem der beiden vorhergehenden Ansprüche, bei der die äußere Verbindungsplatte (26) auf lösbare Weise mit dem Körper (28) der Prothese (10) verbunden ist.

10. Kondylärprothese (10) nach dem vorhergehenden Anspruch, bei der die äußere Verbindungsplatte (26) einen ringartigen Teil (34) für die Befestigung am Körper (28) der Prothese aufweist, und bei der der Körper (28) der Prothese eine zylindrische (36) Auflagefläche für die Aufnahme des ringförmigen Teils (34) der äußeren Verbindungsplatte (26) aufweist, wobei sich die Verbindungsplatte (26) unter dem ringförmigen Teil (34) erstreckt und sich die zylindrische Auflagefläche (36) zwischen dem Gelenkkopf (16) und dem Verankerungsstift (14) befindet und die äußere Oberfläche (S36) der zylindrischen Auflagefläche (36) und die innere Oberfläche (S34) des ringförmigen Teils (34) wenigstens eine Vertiefung (38) und eine Erhöhung (40) aufweisen, die dazu eingerichtet sind, miteinander zusammenzuwirken, um die äußere Verbindungsplatte (26) gegen eine Drehung um den Körper (28) der Prothese festzulegen.

11. Kondylärprothese (10) nach einem der vier vorhergehenden Ansprüche, bei der sich die äußere Verbindungsplatte (26) in einer lateralen Ebene (PL) befindet, die um einen Abstand (D) ungleich Null bezüglich der medialen Ebene (PM) der Prothese in der frontalen Ebene (PF) der Prothese versetzt ist.

12. Kondylärprothese (10) nach einem der fünf vorhergehenden Ansprüche, bei der die äußere Verbindungsplatte (26) im Wesentlichen die Form eines L aufweist, dessen vertikaler Schenkel (42) sich vom Gelenkkopf (16) im Wesentlichen in die gleiche Richtung fortsetzt wie der Verankerungsstift (14) und dessen horizontaler Schenkel (44) sich in die Krümmungsrichtung (DC) des Verankerungsstifts (14) in der medialen Ebene (PM) der Prothese fortsetzt, wobei sich die Durchführungen (31) in dem horizontalen Schenkel (44) des L befinden.

13. Kondylärprothese (10) nach dem vorhergehenden Anspruch, bei der die Länge (L26) der Verbindungsplatte (26) größer als die Länge (L14) des Verankerungsstifts (14) ist, und bei der der horizontale Schenkel (44) des L und somit die Durchführungen (31) unterhalb des Verankerungsstifts (14) angeordnet sind.

14. Kondylärprothese (10) nach Anspruch 12, bei der die Länge (L26) der Verbindungsplatte (26) kleiner oder gleich der Länge (L14) des Verankerungsstifts (14) ist und bei der die Durchführungen (31) zum Ende (46) des horizontalen Schenkels (44) des L hin, der sich nach vorne zum Verankerungsstift (14) erstreckt, ausgebildet sind.

15. Kondylärprothese (10) nach Anspruch 12, bei der die Länge (L26) der Verbindungsplatte (26) im Wesentlichen gleich der Länge (L14) des Verankerungsstifts (14) ist, wobei sich der horizontale Schenkel (44) des L sich nach vorne und nach hinten bezüglich des Verankerungsstifts (14) erstreckt und wobei die Durchführungen (31) in dem horizontalen Schenkel (44) des L ausgebildet sind, so dass diese auf diese Weise nach vorne und hinten bezüglich des Verankerungsstifts (14) versetzt sind.

16. Kondylärprothese (10) nach einem der Ansprüche 7 bis 15, bei der die Dicke (e) der Verbindungsplatte (26) weniger als 0,8 Millimeter beträgt.

17. Kondylärprothese (10) nach einem der vorhergehenden Ansprüche, bei der der Verankerungsstift (14) von einem kugelförmigen Gelenkkopf (16) überragt ist.

18. Kondylärprothese (10) nach einem der vorhergehenden Ansprüche, bei der der Körper (28) des Kieferimplantats (12) aus zwei Teilen hergestellt ist, wobei der Gelenkkopf (16) auf dem Verankerungsstift (14) des Kieferimplantats (12) angebracht ist.

19. Kondylärprothese (10) nach dem vorhergehenden Anspruch, bei der der Gelenkkopf (16) einen Einsetzstift (60) aufweist, und bei dem der Verankerungsstift (14) eine Aufnahme (62) für diesen Einsetzstift (60) aufweist.

20. Vollständige Prothese (50) für ein Kiefergelenk, **dadurch gekennzeichnet, dass** diese eine temporale Prothese (52) und eine Kondylärprothese (10) nach einem der vorhergehenden Ansprüche aufweist.

21. Vollständige Prothese (50) nach dem vorhergehenden Anspruch, bei der der Gelenkkopf (16) aus einem Material hergestellt ist, das es gestattet, die Reibung zu verringern und bei dem die innere Oberfläche (64) der temporalen Prothese (52) wenigstens teilweise mit einer Beschichtung (66) eines Materials abgedeckt ist, das es gestattet, Reibungen zu verringern.

## Claims

1. A condylar prosthesis (10) for a temporomandibular joint, the condylar prosthesis (10) comprising a mandibular implant (12) intended to be secured to the branch (20) of a mandible (22) and an articulation head (16) intended to serve as a substitute mandibular condyle, the mandibular implant (12) comprising an anchoring tail (14) allowing the implementation of an intramedullary connection in the top (18) of the branch (20) of the mandible (22), and the distal end (24) of the anchoring tail (14) being offset towards the front (AV) of the prosthesis (10) with respect to the articulation head (16) and in a midplane (PM) of the prosthesis (10), the condylar prosthesis (10) being **characterised in that** the anchoring tail (14) follows a curved profile (PC) in its length (L14) and in the midplane (PM) of the prosthesis, the direction of curvature (DC) of the anchoring tail (14) being oriented towards the front (AV) of the prosthesis.

2. A condylar prosthesis (10) according to the preceding claim, in which the anchoring tail (14) follows a straight profile (PD) in its length (L14) and in a frontal plane (PF) of the prosthesis perpendicular to the midplane (PM).

3. A condylar prosthesis (10) according to one of the preceding claims, in which the cross section (S14) of the anchoring tail (14) decreases in its length (L14) and towards its distal end (24).

4. A condylar prosthesis (10) according to one of the preceding claims, in which the cross section (S14) of the anchoring tail (14) is non-circular in all or part of its length (L14).

5. A condylar prosthesis (10) according to the preceding claim, in which the cross section (S14) of the anchoring tail (14) is of the elliptical type in all or part of its length (L14), the major axis of the ellipsis remaining parallel to the frontal plane (PF) of the prosthesis in the length (L14) of the anchoring tail (14), and the minor axis of the ellipsis remaining parallel to the midplane (PM) of the prosthesis in the length (L14) of the anchoring tail (14).

6. A condylar prosthesis (10) according to one of the preceding claims, in which the anchoring tail (14) has a rough or granular external surface (SE) in order to increase the intramedullar connection surface and to promote osteointegration of the mandibular implant (12) in the mandibular branch (20).

7. A condylar prosthesis (10) according to one of the preceding claims, in which the mandibular implant (12) also comprises an external connecting plate (26) secured to the body (28) of the prosthesis (10) formed by the anchoring tail (14) and the articulation head (16), this external connecting plate (26) comprising at least two emerging piercings (31) for surgical screws (32) to pass and produced around axes (A31) offset with respect to the anchoring tail (14) both in the frontal plane (PF) of the prosthesis and in the midplane (PM) of the prosthesis.

8. A condylar prosthesis (10) according to the preceding claim, in which the external connecting plate (26) can be shaped.

9. A condylar prosthesis (10) according to one of the preceding two claims, in which the external connecting plate (26) is removably connected to the body (28) of the prosthesis (10).

10. A condylar prosthesis (10) according to the preceding claim, in which the external connecting plate (26) comprises a part of the annular type (34) for connection to the body (28) of the prosthesis, and in which the body (28) of the prosthesis comprises a cylindrical length (36) for receiving the annular part (34) of the external connecting plate (26), the connecting plate (26) extending under the annular part (34), the cylindrical length (36) being situated between the articulation head (16) and the anchoring tail (14), and the external surface (S36) of the cylindrical length (36) and the internal surface (S34) of the annular part (34) comprising at least one hollow recess (38) and a shape in relief (40) able to cooperate with each other in order to immobilise the external connecting plate (26) with respect to rotation about the body (28) of the prosthesis.

11. A condylar prosthesis (10) according to one of the preceding four claims, in which the external connecting plate (26) is situated in a lateral plane (PL) offset by a non-zero distance (D) with respect to the midplane (PM) of the prosthesis in the frontal plane (PF) of the prosthesis.

12. A condylar prosthesis (10) according to one of the preceding five claims, in which, the external connecting plate (26) taking substantially the form of an L, the vertical branch (42) of which extending from the articulation head (16) substantially in the same direction as the anchoring tail (14), and the horizontal branch (44) of which extending in the direction of curvature (DC) of the anchoring tail (14) in a midplane (PM) of the prosthesis, the emerging piercings (31) being situated in the horizontal branch (44) of the L.

13. A condylar prosthesis (10) according to the preceding claim, in which the length (L26) of the connecting plate (26) is greater than the length (L14) of the anchoring tail (14), and in which the horizontal branch (44) of the L, and therefore the emerging piercings (31), are offset below the anchoring tail (14).

14. A condylar prosthesis (10) according to claim 12, in which the length (L26) of the connecting plate (26) is less than or equal to the length (L14) of the anchoring tail (14), and in which the emerging piercings (31) are produced towards the end (46) of the horizontal branch (44) of the L extending in front of the anchoring tail (14).

15. A condylar prosthesis (10) according to claim 12, in which, the length (L26) of the connecting plate (26) being substantially equal to the length (L14) of the anchoring tail (14), the horizontal branch (44) of the L extends in front of and behind the anchoring tail (14), and the emerging piercings (31) are produced in the horizontal branch (44) of the L so as to be offset in front of and behind the anchoring tail (14).

16. A condylar prosthesis (10) according to any of claims 7 to 15, in which the thickness (e) of the connecting plate (26) is less than 0.8 millimetres.

17. A condylar prosthesis (10) according to any of the preceding claims, in which the anchoring tail (14) is surmounted by a spherical articulation head (16).

18. A condylar prosthesis (10) according to one of the preceding claims, in which the body (28) of the mandibular implant (12) is produced in two parts, the articulation head (16) being attached to the anchoring tail (14) of the mandibular implant (12).

19. A condylar prosthesis (10) according to the preceding claim, in which the articulation head (16) comprises a fitting-in tail (60), and in which the anchoring tail (14) comprises a housing (62) for receiving this fitting-in tail (60).

20. A complete prosthesis (50) for a temporomandibular articulation, **characterised in that** it comprises a temporal prosthesis (52) and a condylar prosthesis (10) according to any of the preceding claims.

21. A complete prosthesis (50) according to the preceding claim, in which the articulation head (16) is manufactured from a material making it possible to reduce friction, and in which the bottom surface (64) of the temporal prosthesis (52) is covered, at least partly, by a cladding (66) of material for reducing friction.
